# FASCICULE DE BREVET EUROPEEN

(11) **EP 0 692 237 B1**
(45) Date de publication et mention de la délivrance du brevet: **26.06.1996**
(21) Numéro de dépôt: 95401114.4
(22) Date de dépôt: 12.05.1995
(51) Int. Cl.: A61K 7/00

(54) **Emulsion huile-dans-eau sans tensioactif, stabilisée par des particules thermoplastiques creuses**
Öl-in-Wasser Emulsion ohne grenzflächenaktiven Stoff, die durch hohle thermoplastische Partikel stabilisiert ist
Surface active agents free oil in water emulsion, stabilized by hollow thermoplastic particles

(30) Priorité: 11.07.1994 FR 9408561
(43) Date de publication de la demande: 17.01.1996
(73) Titulaire: L'OREAL, F-75008 Paris (FR)
(72) Inventeur: Bara, Isabelle, F-75013 Paris (FR); Touzan, Philippe, F-75012 Paris (FR)
(74) Mandataire: Lhoste, Catherine

(56) Documents cités:
- EP-A- 0 502 769
- FR-A- 2 700 952
- GB-A- 2 191 945

## Description

L'invention se rapporte à une émulsion huile-dans-eau (H/E) ne comportant pas de tensioactif. Cette émulsion se présente sous forme d'une crème ou d'un lait, blancs ou colorés, destinés notamment au traitement cosmétique de la peau (corps, visage) et/ou des cheveux. Cette crème peut, en outre, servir au maquillage, ou encore au traitement dermatologique de la peau et/ou des cheveux.

Pour diverses raisons liées en particulier à leur grand confort d'utilisation et à leur fraicheur, les émulsions du type huile-dans-eau sont aujourd'hui largement utilisées dans le domaine cosmétique ou dermatologique. Ces émulsions comportent une phase huileuse dispersée dans une phase aqueuse et un ou plusieurs tensioactifs qui stabilisent la dispersion obtenue. Elles présentent l'inconvénient de contenir des tensioactifs ; or, on sait que les tensioactifs peuvent se montrer irritants pour certains types de peau. Aussi, cherche-t-on de plus en plus à s'en affranchir.

Par ailleurs, la présence de tensioactifs nécessite le plus souvent la fabrication de l'émulsion à chaud, ce qui limite notamment la nature des actifs à introduire dans l'émulsion. En particulier, ce procédé exclut l'emploi d'actifs thermosensibles.

Il subsiste donc le besoin d'une émulsion H/E ne présentant pas les inconvénients de celles connues jusqu'à présent et, notamment, ne contenant pas de tensioactif.

La présente invention a donc pour objet une émulsion d'une phase huileuse dans une phase aqueuse gélifiée, caractérisée en ce qu'elle contient des particules thermoplastiques creuses expansées de polymère ou copolymère d'acrylonitrile assurant la dispersion de la phase huileuse dans la phase aqueuse, et en ce qu'elle est exempte de tensioactif. Elle contient, en outre, avantageusement un milieu cosmétiquement et/ou pharmaceutiquement acceptable.

L'émulsion revendiquée présente l'avantage de ne pas contenir de tensioactif, donc de ne pas être irritante, et aussi d'être très douce à l'application grâce à la présence des particules creuses.

En outre, l'utilisation des particules creuses pour stabiliser l'émulsion permet de la fabriquer à froid, ce qui est plus simple et moins coûteux que le procédé classique réalisé le plus souvent à chaud quand on utilise des tensioactifs. La fabrication à froid permet par exemple l'introduction d'actifs thermosensibles sans risque de dégradation de ces actifs.

De plus, l'utilisation des particules creuses permet d'obtenir une composition de texture particulière très agréable, qui n'est pas grasse malgré la présence d'huiles, et qui n'est pas collante malgrè la présence d'agents gélifiants.

L'invention a aussi pour objet une utilisation de l'émulsion définie ci-dessus pour le traitement cosmétique de la peau (par exemple nutrition, hydratation, protection, nettoyage, maquillage) et/ou des cheveux (protection, lissage) ainsi que pour la préparation d'une crème destinée au traitement des maladies de la peau (par exemple mycoses, acné, inflammations et psoriasis) et/ou des cheveux.

L'invention a encore pour objet un procédé de traitement cosmétique consistant à appliquer sur la peau et/ou les cheveux l'émulsion définie ci-dessus.

Certes, il est connu par l'article de C. Levine (Colloids and surfaces, 1989, volume 38, pages 325 à 343, "Stabilization of emulsions by fine particules I. Partitioning of particles between continuous phase and oil/water interface") d'utiliser des particules fines de silice, rendues hydrophobes par traitement de surface, pour stabiliser des dispersions ne contenant pas de tensioactif, mais ce document ne permet pas de conduire l'homme du métier du domaine cosmétique à l'invention, d'une part parce qu'il concerne un domaine technique tout à fait différent, celui des huiles lourdes et des bitumes, et, d'autre part, parce que l'enseignement qui y est donné ne conduit pas à un système homogène, mais à des dispersions grossières dont les gouttelettes sont visibles à l'oeil nu. Or, par définition, une émulsion cosmétique est une dispersion homogène de deux phases l'une dans l'autre.

De plus, les particules qui y sont utilisées sont de par leur nature différentes de celles qui permettent d'obtenir l'émulsion selon l'invention. Dans la présente invention, on cherche à obtenir une émulsion qui soit la moins irritante possible et qui ait de bonnes qualités sensorielles, et notamment qui soient très douces à l'application.

Il est par ailleurs connu selon le document FR-A-2208642 une composition cosmétique liquide à deux phases distinctes, l'une étant constituée d'eau et de solvant organique (éthanol ou propanol) et l'autre étant constituée d'huile, des particules finement divisées étant situées à l'interface des deux phases. Les particules sont constituées de substances minérales ou de substances organiques synthétiques telles que le chlorure de polyvinyle. Ces particules sont destinées à faciliter la formation des billes d'huile dans le milieu hydroalcoolique.

Du fait du type de produit obtenu, à savoir une composition à deux phases, et du fait de la présence de solvant consistant en un monoalcool, ce document ne pouvait pas conduire l'homme du métier à utiliser des particules creuses thermoplastiques pour stabiliser une émulsion huile-dans-eau ne comportant pas de monoalcool. Dans la présente invention, on cherche à obtenir une émulsion homogène, la moins irritante possible, donc sans monoalcool, et non une composition biphasique contenant de l'alcool ou des tensioactifs qui peuvent se révéler irritants.

Il est de plus connu selon le document EP-392426 d'utiliser des particules en polyméthacrylate de méthyle pour introduire dans un système gélifié un actif tel qu'une huile, qui est adsorbé sur les particules.

Mais ce type de particules ne permet d'introduire qu'une petite quantité d'huile dans un gel, et pas d'obtenir la dispersion d'une phase huileuse dans une phase aqueuse, comme le montre le test comparatif indiqué ci-dessous.

Aussi, l'invention a encore pour objet l'utilisation de particules thermoplastiques creuses expansées de polymère ou copolymère d'acrylonitrile pour disperser, sans tensio-actif, une phase huileuse dans une phase aqueuse gélifiée et stabiliser l'émulsion obtenue.

De façon générale, les particules utilisables dans l'invention peuvent être réalisées en tout polymère ou copolymère d'acrylonitrile, expansé, non toxique et non irritant pour la peau.

En particulier, la masse volumique des particules est choisie dans la gamme allant de 15 kg/m³ à 200 kg/m³ et mieux de 40 kg/m³ à 120 kg/m³, et encore mieux de 60 kg/m³ à 80 kg/m³. Pour obtenir cette faible masse volumique, on utilise avantageusement des particules de polymères ou copolymères expansés de préférence à base d'acrylonitrile et d'un monomère acrylique ou styrénique et/ou de chlorure de vinylidène.

On peut par exemple utiliser un copolymère contenant: de 0 % à 60 % de motifs dérivés du chlorure de vinylidène, de 20 % à 90 % de motifs dérivés d'acrylonitrile et de 0 % à 50 % de motifs dérivés d'un monomère acrylique ou styrénique, la somme des pourcentages (en poids) étant égale à 100. Le monomère acrylique est par exemple un acrylate ou méthacrylate de méthyle ou d'éthyle. Le monomère styrénique est par exemple l'α-méthyl-styrène ou le styrène.

De préférence, les particules utilisées dans la présente invention sont des particules creuses d'un copolymère expansé de chlorure de vinylidène et d'acrylonitrile ou de chlorure de vinylidène, d'acrylonitrile et de méthacrylate. Ces particules peuvent être sèches ou hydratées.

Les particules de l'invention peuvent être obtenues, par exemple, selon les procédés des brevets et demandes de brevet EP-56219, EP-348372, EP-486080, EP-320473, EP-112807 et US-3615972.

La cavité interne des particules contient en principe un gaz qui peut être de l'air, de l'azote ou un hydrocarbure comme l'isobutane ou l'isopentane.

De façon avantageuse, les particules de l'invention ont une granulométrie allant de 1 µm à 80 µm et encore mieux allant de 10 µm à 50 µm, et encore mieux de 10 µm à 30 µm.

Les particules utilisables dans l'invention sont par exemple les microsphères de copolymère expansé de chlorure de vinylidène, d'acrylonitrile et de méthacrylate, vendues sous la marque EXPANCEL par la société Nobel Casco sous les références 551 DE 50 (granulométrie d'environ 40 µm), 551 DE 20 (granulométrie d'environ 30 µm et masse volumique d'environ 65 kg/m³), 551 DE 12 (granulométrie d'environ 12 µm), 551 DE 80 (granulométrie d'environ 80 µm), 461 DE 50 (granulométrie d'environ 50 µm). On peut aussi utiliser des microsphères formées du même terpolymère expansé ayant une granulométrie d'environ 18 µm et une masse volumique d'environ 70 kg/m³, appelées ci-dessous EL 23, ou ayant une granulométrie d'environ 34 µm et une masse volumique d'environ 20 kg/m³, appelées ci-dessous EL 43.

Ces particules confèrent en outre aux émulsions les contenant de la légèreté et de la matité à l'application.

Dans les compositions de l'invention, on utilise de préférence de 0,1 % à 10 % en poids de particules dans la composition, par rapport au poids total de la composition, et mieux de 0,5 % à 5 % en poids.

Les émulsions selon l'invention comportent une phase aqueuse gélifiée à l'aide d'au moins un agent gélifiant, notamment un agent gélifiant hydrophile, de telle sorte que la viscosité de la phase aqueuse continue soit suffisamment élevée pour permettre une bonne stabilité de l'émulsion dans le temps et éviter que les particules, dont la densité est extrèmement basse, ne remontent à la surface et ne provoquent ainsi une déstabilisation de l'émulsion.

L'agent ou les agents gélifiants peuvent être choisis notamment parmi les polymères carboxyvinyliques (carbomer) tels que ceux vendus sous les dénominations Carbopol par la société Goodrich les polyacrylates et les polyméthacrylates tels que les produits vendus sous les dénominations de Lubrajel ou Norgel par la société Guardian ou sous la dénomination Hispagel par la société Hispano Chimica ; les polyacrylamides tels que le produit vendu sous la dénomination Sepigel 305 par la société Seppic ; les polysaccharides tels que les alginates, la cellulose et ses dérivés, notamment la carboxyméthylcellulose, l'hydroxyméthylcellulose, l'hydroxypropylcellulose et la cellulose microcristalline ; les gommes naturelles telles que la gomme de xanthane, la gomme de guar, la gomme de caroube, la gomme d'acacia, les scléroglucanes, les dérivés de chitine et de chitosane, les carraghénanes ; ou les argiles telles que la montmorillonite, les bentones et les silicates d'aluminium et magnésium (Veegum).

La quantité de gélifiant utilisée dans l'émulsion de l'invention dépend de la nature du ou des gélifiants utilisés. Elle peut par exemple aller de 0,1 % à 10 % en poids, et de préférence de 0,2 % à 6 % en poids par rapport au poids total de l'émulsion.

La phase huileuse de l'émulsion peut représenter de 0,1 à 30 % en poids, et mieux de 5 à 15 % en poids par rapport au poids total de l'émulsion et comprendre des huiles et éventuellement d'autres corps gras.

Comme huiles utilisables dans l'invention, on peut citer par exemple les huiles minérales, les huiles d'origine animale, les huiles végétales, les huiles synthétiques, les huiles siliconées et les huiles fluorées.

Parmi les huiles minérales, on peut citer par exemple l'huile de paraffine, l'huile de vaseline et les huiles minérales ayant un point d'ébullition entre 300 et 400°C.

Parmi les huiles d'origine animale, on peut citer par exemple le perhydrosqualène.

Parmi les huiles végétales, on peut citer par exemple l'huile d'amande douce, l'huile de calophyllum, l'huile de palme, l'huile de noyau d'abricot, l'huile d'avocat, l'huile de jojoba, l'huile d'olive, l'huile de ricin, les huiles de germes de céréales (huile de germes de maïs), la fraction liquide de beurre de karité.

Parmi les huiles synthétiques, on peut citer par exemple le polyisobutène hydrogéné ; les esters d'acides gras tels que l'huile de purcellin, le myristate de butyle, le myristate d'isopropyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate de décyle, le laurate d'hexyle, le dicaprylate de propylèneglycol, les esters dérivés d'acide lanolique tels que le lanolate de disopropyle, le lanolate d'isocétyle les acétylglycérides les octanoates et décanoates d'alcool et de polyalcools tels que ceux de glycol et de glycérol ; les ricinoléates d'alcools et de polyalcools.

Parmi les huiles siliconées, on peut citer par exemple les cyclométhicones, les polydiméthylsiloxanes de faible poids moléculaire (huile de silicone) ou de haut poids moléculaire (gomme de silicone), les polyméthylsiloxanes, les diméthiconols, les polydiméthylsiloxanes phénylées, les siloxanols de faible poids moléculaire et de haut poids moléculaire, et les triméthylsiloxysilicates.

Parmi les huiles fluorées, on peut citer par exemple les perfluoroéthers et les silicones fluorées.

Les autres corps gras susceptibles d'être présents dans la phase huileuse peuvent être par exemple les acides gras, les alcools gras et les cires.

En outre, de façon connue, les émulsions de l'invention peuvent contenir des adjuvants habituels dans le domaine cosmétique ou dermatologique, tels que des actifs hydrophiles ou lipophiles, des conservateurs, des antioxydants, des parfums, des charges, des filtres solaires, des matières colorantes (pigments et colorants) et encore des vésicules lipidiques. Ces adjuvants sont utilisés dans les proportions habituelles des émulsions utilisées dans le domaine cosmétique ou dermatologique, et par exemple de 0,01 % à 20 % en poids par rapport au poids total de l'émulsion, et ils sont, selon leur nature, introduits dans la phase aqueuse gélifiée ou dans la phase huileuse de l'émulsion.

On peut citer notamment comme actifs hydrophiles les protéines ou les hydrolysats de protéine, les acides aminés (hydroxyproline, proline), les polyols tels que la glycérine, le sorbitol, le butylène glycol, le propylène glycol ou le polyéthylène glycol, l'allantoïne, la guanosine, les sucres et dérivés de sucre, les vitamines hydrosolubles telles que l'acide ascorbique (vitamine C), les hydroxyacides et leurs sels et des actifs spécifiques hydrosolubles tels que les actifs hydratants, anti-rides, amincissants, nutritifs, adoucissants, etc.

Comme actifs lipophiles, on peut par exemple citer les vitamines liposolubles telles que le rétinol (vitamine A) et ses dérivés (palmitate de rétinyle), le tocophérol (vitamine E) et ses dérivés ; les filtres solaires ; les céramides et les actifs spécifiques liposolubles tels que les actifs amincissants, les agents anti-radicaux libres.

Il va de soi que la liste donnée ci-dessus des actifs hydrophiles ou lipophiles n'est nullement exhaustive.

Les émulsions objet de l'invention trouvent leur application dans un grand nombre de traitements cosmétiques et/ou dermatologiques de la peau et des cheveux, y compris le cuir chevelu, notamment pour le soin, le nettoyage et le maquillage de la peau, pour le soin des cheveux et pour le traitement thérapeutique de la peau, des cheveux et des muqueuses.

Ces émulsions peuvent par exemple être utilisées dans des produits de soin ou de maquillage du visage pour peaux sèches ou peaux grasses.

Ainsi, par exemple, pour la réalisation de produits pour peaux sèches, on peut introduire dans la phase aqueuse gélifiée, des substances actives hydrosolubles hydratantes telles que par exemple la glycérine, le propylène glycol, le sorbitol, la proline, l'acide pyrrolidone carboxylique et ses dérivés, l'urée, le collagène hydrolysé, le gel d'Aloe Vera, l'acide hyaluronique et ses dérivés, le hyaluronate de diméthylsilanol, l'allantoïne, le D-panthénol et le lactate de sodium.

En revanche, des produits de traitement pour peaux grasses sont notamment obtenus en introduisant dans la phase aqueuse gélifiée des substances actives hydrosolubles telles que la provitamine B5 qui est utilisée comme adoucissant, ou un antibactérien tel que le transthiolane diol 3,4-S-dioxyde.

Les émulsions selon l'invention peuvent également être utilisées comme produits démaquillants ou de nettoyage pour le visage sous forme de crèmes, de laits ou de masques par exemple, ou comme produits de maquillage par incorporation de charges, de pigments ou de colorants. On peut obtenir notamment des produits pour le teint, des fards à joues, des fards à paupières, des fonds de teint, des crèmes teintées, des produits colorants pour le corps tels que les produits teintés donnant un effet de hâle naturel.

Les émulsions de l'invention peuvent aussi être utilisées comme produits solaires par introduction de filtres hydrosolubles ou liposolubles pour protéger la peau et/ou les cheveux des rayonnements UV. Comme filtres solaires hydrosolubles pouvant être introduits dans la phase aqueuse gélifiée, on peut citer par exemple l'acide 2-hydroxy 4-méthoxy benzophénone 5-sulfonique vendu sous la dénomination UVINUL MS 40 par la Société BASF. Comme filtres solaires liposolubles à introduire dans la phase huileuse, on peut citer par exemple le paraméthoxycinnamate de 2-éthylhexyle vendu sous la dénomination PARSOL MCX par la Société GIVAUDAN ou la 2-hydroxy 4-méthoxybenzophénone vendue sous la dénomination UVINUL M 40 par la Société BASF.

Les émulsions selon l'invention peuvent également être utilisées dans la préparation de produits après-solaires contenant par exemple, en tant qu'agents actifs apaisants, la vitamine F et les hydratants cités plus haut.

On peut aussi obtenir des produits amincissants en introduisant des substances actives amincissantes hydrosolubles ou liposolubles respectivement dans la phase aqueuse gélifiée ou dans la phase huileuse. Parmi les substances actives amincissantes hydrosolubles, on peut citer les dérivés de xanthine tels que la caféine, la théobromine, la théophylline, la L-carnitine, le chlorhydrate de diméthylaminoéthyl théophylline ; les dérivés de silicium du type méthylsilanol théophyllinacétate alginate ou des dérivés végétaux tels que des extraits hydroglycoliques de lierre grimpant, d'algue brune, de pensée sauvage fraîche. Parmi les substances actives amincissantes liposolubles, on peut citer le nicotinate de DL-alpha-tocophérol, l'extrait huileux de racine de ginseng (*Panax ginseng*), l'extrait huileux de lierre grimpant (*Hedera Helix*), l'extrait huileux de fleurs sèches d'arnica (*Arnica Montana L*), l'extrait huileux d'algue (*Fucus Vesiculosus*).

Les émulsions de l'invention peuvent également être utilisées comme produits pour jambes lourdes contenant comme actifs hydrosolubles le ginkgo biloba, le mélilot ou le ruscus et des huiles classiquement utilisées comme émollients.

Enfin, les émulsions selon l'invention peuvent être utilisées également en dermatologie, par exemple pour le soin des peaux sensibles ou irritées, pour le traitement du psoriasis, de l'eczéma, de l'acné ou des mycoses, après introduction dans l'émulsion d'actifs appropriés, tels que des anti-inflammatoires, des antifongiques, des antiparasitaires, des agents antiprurugineux, des agents antiséborrhéiques, des agents kératolytiques ou des agents anti-acnéiques.

De manière générale, les émulsions selon l'invention peuvent être préparées selon les procédés à froid connus dans les domaines cosmétique et dermatologique. On peut par exemple d'abord préparer un gel aqueux en mélangeant le gélifiant et l'eau, puis y ajouter les actifs et autres adjuvants hydrophiles, et ensuite y disperser les particules creuses. Puis, on émulsionne la phase grasse contenant les huiles et corps gras, les actifs et autres adjuvants hydrophobes, dans ce gel aqueux sous agitation durant quelques minutes à la température ambiante. Le stade de préparation du gel est susceptible de nécessiter un peu de chauffage, mais les actifs thermosensibles sont toujours ajoutés à froid.

Le produit obtenu est un gel-crème qui présente d'excellentes propriétés cosmétiques, notamment en ce qui concerne le toucher et l'aspect, permettant son utilisation comme base pour produits cosmétiques.

La demanderesse a testé des particules de nature différente dans une émulsion huile-dans-eau en vue de comparer leur effet dispersant. La composition de l'émulsion était la suivante :

| | |
|---|---|
| - Cyclométhicone | 10 % |
| - Carbomer (gélifiant) | 0,8 % |
| - Triéthanolamine (neutralisant) | 0,8 % |
| - Particules | 1 % |
| - Eau | qsp 100 % |

Les résultats sont donnés dans le tableau ci-dessous. On constate que seules les particules d'Expancel assurent une bonne dispersion de la phase huileuse dans la phase aqueuse.

| **Type de charge** | **Résultats Aspect et stabilité** |
|---|---|
| Polytrap 6603 (polyméthacrylate de méthyle) ; aggrégats de 200 µm à 1200 µm. Densité : 0,06 | Dispersion grossière. Relargage d'huile en surface en centrifugation |
| Expancel EI 23 | Crème ivoire et brillante. Bonne stabilité en centrifugation |

Les exemples suivants illustrent l'invention. Dans ces exemples, les pourcentages sont donnés en poids.

### Exemple 1: Soin contour des yeux

| *Phase A1* | |
|---|---|
| Guanosine | 0,01 % |
| Hydroxyproline | 1,00 % |
| Conservateurs | 0,30 % |
| Eau déminéralisée | qsp 100 % |

| *Phase A2* | |
|---|---|
| Eau déminéralisée | 22,00 % |
| Glycérine | 10,00 % |
| Carbomer (gélifiant) | 0,80 % |

| *Phase A3* | |
|---|---|
| Triéthanolamine (neutralisant) | 0,80 % |

| *Phase B* | |
|---|---|
| Microsphères EL 23 | 1,30 % |

| *Phase C* | |
|---|---|
| Huile de germes de maïs | 10,00 % |
| Palmitate de rétinyle | 0,15 % |
| Parfum | 0,30 % |

On prépare la phase A1 en dissolvant la guanosine dans l'eau, puis en y ajoutant l'hydroxyproline et les conservateurs à température ambiante.

On prépare par ailleurs la phase A2 en faisant gonfler le carbomer, sous agitation à la turbine, à la température ambiante dans l'eau additionnée de glycérine. Puis on introduit la phase A3 dans la phase A2.

La phase C est préparée en mélangeant les constituants à la température ambiante.

La phase A1 et et le mélange des phases A2 et A3 sont homogénéisés. On y disperse ensuite sous agitation la phase B, puis on ajoute la phase C.

Après agitation sous turbine durant 15 min., on obtient un gel-crème destiné au soin du contour de l'oeil.

Le produit obtenu est très doux à l'application et sa formule reste bien stable au cours du temps.

### Exemple 2: Fluide après-rasage

| *Phase A1* | |
|---|---|
| Guanosine | 0,01 % |
| Allantoïne | 0,20 % |
| Conservateurs | 0,30 % |
| Eau déminéralisée | qsp 100 % |

| *Phase A2* | |
|---|---|
| Eau déminéralisée | 22,00 % |
| Glycérine | 7,00 % |
| Carbomer (gélifiant) | 0,40 % |
| Gomme de xanthane (gélifiant) | 0,10 % |

| *Phase A3* | |
|---|---|
| Triéthanolamine (neutralisant) | 0,40 % |

| *Phase B* | |
|---|---|
| Microsphères EL 23 | 0,80 % |

| *Phase C* | |
|---|---|
| Cyclométhicone | 8,00 % |
| Parfum | 0,50 % |

Le mode opératoire est le même que dans l'exemple 1. On obtient un fluide après-rasage qui est onctueux et non irritant, et dont le toucher est très doux.

### Exemple 3: Baume corporel hydratant

| *Phase A1* | |
|---|---|
| D-panthénol | 1,0 % |
| Urée | 2,0 % |
| Lactate de sodium | 1,0 % |
| Conservateurs | 0,4 % |
| Eau déminéralisée | qsp 100 % |

| *Phase A2* | |
|---|---|
| Eau déminéralisée | 25,0 % |
| Glycérine | 5,0 % |
| Carbomer (gélifiant) | 0,8 % |
| Gomme de xanthane (gélifiant) | 0,1 % |

| *Phase A3* | |
|---|---|
| Triéthanolamine (neutralisant) | 0,8 % |

| *Phase B* | |
|---|---|
| Microsphères EL23 | 1,0 % |

| *Phase C* | |
|---|---|
| Fraction liquide de beurre de karité | 8,0 % |
| Parfum | 0,3 % |

Le mode opératoire est le même que dans l'exemple 1. On obtient un baume corporel très doux dont l'application est très agréable et qui hydrate bien la peau sans provoquer d'irritation.

### Exemple 4: Masque nettoyant doux

| *Phase A* | |
|---|---|
| Gomme d'acacia (gélifiant) | 0,62 % |
| Carraghénane (gélifiant) | 1,50 % |
| Gomme de xanthane (gélifiant) | 0,25 % |
| Montmorillonite (gélifiant) | 2,00 % |
| Butylène glycol | 3,00 % |
| Conservateur | qs |
| Eau déminéralisée | qsp 100 % |

| *Phase B* | |
|---|---|
| Amidon de froment (charge) | 2,00 % |
| Dioxyde de titane (pigment) | 2,00 % |
| Oxyde de fer jaune (pigment) | 0,03 % |
| EXPANCEL 551 DE 20 | 6,00 % |

| *Phase C* | |
|---|---|
| Huile de paraffine | 5,00 % |

Le mode opératoire consiste à préparer la phase A en dispersant tous les constituants dans l'eau à la température ambiante, puis à empâter la phase B dans la phase A et à incorporer ensuite la phase grasse en agitant fortement pendant 10 min.

Le masque obtenu a une texture très légère. Il est très doux à l'application et ne dessèche pas la peau.

### Exemple 5: Fard à joues

| *Phase A* | |
|---|---|
| Carbomer (gélifiant) | 0,60 % |
| Triéthanolamine (neutralisant) | 0,60 % |
| Conservateurs | qs |
| Glycérine | 2,00 % |
| Jaune de quinoléine (colorant) | 0,07 % |
| Sel disodique de ponceau (colorant) | 0,09 % |
| Sel disodique du vert d'alizarine (colorant) | 0,04 % |
| Eau déminéralisée | qsp 100 % |

| *Phase B* | |
|---|---|
| Cyclométhicone/diméthiconol (mélange vendu sous la dénomination DC 1401 Substantivity Aid Fluid par Dow Corning) | 4,00 % |
| Polyisobutène hydrogéné | 4,00 % |

| *Phase C* | |
|---|---|
| EXPANCEL 551 DE 20 | 0,80 % |

Le mode opératoire consiste à préparer la phase aqueuse gélifiée A à 80°C en mélangeant les différents constituants dans l'eau, puis à la laisser refroidir jusqu'à la température ambiante en agitant. On y ajoute ensuite la phase C sous agitation assez lente et on y incorpore la phase B sous agitation moyenne pendant une dizaine de minutes.

On obtient un fard à joues gélifié, très doux, facile à étaler et qui donne au final un effet poudré très naturel.

### Exemple 6: Gel teinté

| *Phase A* | |
|---|---|
| Carbomer (gélifiant) | 1,50 % |
| Triéthanolamine (neutralisant) | 1,50 % |
| Polyéthylène glycol | 8,40 % |
| Conservateurs | 0,30 % |
| Glycérine | 2,00 % |
| Jaune de quinoléine (colorant) | 0,07 % |
| Sel disodique de ponceau (colorant) | 0,09 % |
| Sel disodique du vert d'alizarine (colorant) | 0,04 % |
| Eau déminéralisée | qsp 100 % |

| *Phase B* | |
|---|---|
| Cyclométhicone/diméthiconol (mélange vendu sous la dénomination DC 1401 Substantivity Aid Fluid par Dow Corning) | 5,00 % |

| *Phase C* | |
|---|---|
| Microsphères EL 23 | 1,00 % |

Le mode opératoire est le même que pour l'exemple précédent.

On obtient un gel teinté pour le visage ou pour le corps, très doux à l'application, facile à étaler et à uniformiser. Ce gel n'est pas gras et n'est pas collant.

### Exemple 7: Crème dermatologique

| *Phase A* | |
|---|---|
| Carbomer (gélifiant) | 6,00 % |
| Soude (neutralisant) | 1,00 % |
| Eau déminéralisée | qsp 100 % |

| *Phase B* | |
|---|---|
| Microsphères EL 23 | 1,00 % |

| *Phase C* | |
|---|---|
| Huile de vaseline fluide | 6,00 % |

| *Phase D* | |
|---|---|
| Propionate de clobetasol 17 (anti-inflammatoire) | 0,01 % |
| Propylène glycol | 5,00 % |

Le mode opéraroire consiste à préparer la phase A, à y introduire la phase B sous agitation modérée, puis à y incorporer la phase C goutte à goutte en maintenant l'agitation, à homogénéiser sous agitation magnétique et enfin à y ajouter la phase D sous agitation.

On obtient une crème très douce à l'application, adaptée au traitement du psoriasis.

## Revendications

1. Emulsion d'une phase huileuse dans une phase aqueuse gélifiée, caractérisée en ce qu'elle contient des particules thermoplastiques creuses expansées de polymère ou copolymère d'acrylonitrile, assurant la dispersion de la phase huileuse dans la phase aqueuse gélifiée, et en ce qu'elle est exempte de tensioactif.

2. Emulsion selon la revendication 1, caractérisée en ce qu'elle est exempte de monoalcool.

3. Emulsion selon la revendication 1 ou 2, caractérisée en ce que la phase aqueuse gélifiée contient au moins un gélifiant choisi parmi les polymères carboxyvinyliques, les polyacrylamides, les polyacrylates, les polyméthacrylates, les polysaccharides, les gommes naturelles et les argiles.

4. Emulsion selon l'une quelconque des revendications 1 à 3, caractérisée en ce que les particules ont une granulométrie de 1 à 80 µm.

5. Emulsion selon l'une quelconque des revendications 1 à 4, caractérisée en ce que les particules ont une granulométrie de 10 à 50 µm.

6. Emulsion selon l'une quelconque des revendications 1 à 5, caractérisée en ce que les particules ont une masse volumique de 15 à 200 kg/m³.

7. Emulsion selon l'une quelconque des revendications 1 à 6, caractérisée en ce que les particules ont une masse volumique de 40 à 120 kg/m³.

8. Emulsion selon l'une quelconque des revendications 1 à 7, caractérisée en ce que les particules sont des particules creuses expansées de copolymères d'acrylonitrile et de chlorure de vinylidène et/ou d'un monomère acrylique ou styrénique.

9. Emulsion selon l'une quelconque des revendications 1 à 8, caractérisée en ce que les particules représentent de 0,1 % à 10 % en poids par rapport au poids total de l'émulsion.

10. Emulsion selon l'une quelconque des revendications 1 à 9, caractérisée en ce que le gélifiant représente de 0,1 % à 10 % en poids par rapport au poids total de l'émulsion.

11. Emulsion selon l'une quelconque des revendications 1 à 10, caractérisée en ce que la phase huileuse représente de 0,1 % à 30 % en poids par rapport au poids total de l'émulsion.

12. Emulsion selon l'une quelconque des revendications 1 à 11, caractérisée en ce qu'elle consiste en une composition cosmétique et/ou dermatologique.

13. Emulsion selon l'une quelconque des revendications 1 à 12, caractérisée en ce qu'elle contient au moins un additif choisi parmi les actifs hydrophiles, les actifs lipophiles, les conservateurs, les antioxydants, les parfums, les charges, les filtres solaires, les matières colorantes et les vésicules lipidiques.

14. Utilisation de l'émulsion selon l'une quelconque des revendications 1 à 13 pour le traitement cosmétique de la peau et/ou les cheveux.

15. Utilisation de l'émulsion selon l'une quelconque des revendications 1 à 13 pour l'obtention d'une crème destinée au traitement dermatologique de la peau.

16. Procédé de traitement cosmétique, caractérisé en ce qu'il consiste à appliquer sur la peau et/ou les cheveux l'émulsion selon l'une quelconque des revendications 1 à 13.

17. Utilisation de particules thermoplastiques creuses expansées de polymère ou copolymère d'acrylonitrile pour disperser, sans tensioactif, une phase huileuse dans une phase aqueuse gélifiée et stabiliser l'émulsion ainsi obtenue.

18. Utilisation selon la revendication 17, caractérisée en ce que les particules ont une granulométrie de 1 à 80 µm.

19. Utilisation selon la revendication 17 ou 18, caractérisée en ce que les particules ont une granulométrie de 10 à 50 µm.

20. Utilisation selon l'une quelconque des revendications 17 à 19, caractérisée en ce que les particules ont une masse volumique de 15 à 200 kg/m³.

21. Utilisation selon l'une quelconque des revendications 17 à 20, caractérisée en ce que les particules ont une masse volumique de 40 à 120 kg/m³.

22. Utilisation selon l'une quelconque des revendications 17 à 21, caractérisée en ce que les particules sont des particules creuses expansées de copolymères d'acrylonitrile et de chlorure de vinylidène et/ou d'un monomère acrylique ou styrénique.

## Claims

1. Emulsion of an oily phase in a gelled aqueous phase, characterized in that it contains expanded hollow thermoplastic particles of acrylonitrile polymer or copolymer, ensuring the dispersion of the oily phase in the gelled aqueous phase, and in that it is free from surfactant.

2. Emulsion according to Claim 1, characterized in that it is free from monoalcohol.

3. Emulsion according to Claim 1 or 2, characterized in that the gelled aqueous phase contains at least one gelling agent chosen from carboxyvinyl polymers, polyacrylamides, polyacrylates, polymethacrylates, polysaccharides, natural gums and clays.

4. Emulsion according to any one of Claims 1 to 3, characterized in that the particles have a particle size of 1 to 80 µm.

5. Emulsion according to any one of Claims 1 to 4, characterized in that the particles have a particle size of 10 to 50 µm.

6. Emulsion according to any one of Claims 1 to 5, characterized in that the particles have a density of 15 to 200 kg/m³.

7. Emulsion according to any one of Claims 1 to 6, characterized in that the particles have a density of 40 to 120 kg/m³.

8. Emulsion according to any one of Claims 1 to 7, characterized in that the particles are expanded hollow particles of copolymers of acrylonitrile and of vinylidene chloride and/or of an acrylic or styrene-based monomer.

9. Emulsion according to any one of Claims 1 to 8, characterized in that the particles represent from 0.1 % to 10 % by weight relative to the total weight of the emulsion.

10. Emulsion according to any one of Claims 1 to 9, characterized in that the gelling agent represents from 0.1 to 10 % by weight relative to the total weight of the emulsion.

11. Emulsion according to any one of Claims 1 to 10, characterized in that the oily phase represents from 0.1 % to 30 % by weight relative to the total weight of the emulsion.

12. Emulsion according to any one of Claims 1 to 11, characterized in that it consists of a cosmetic and/or dermatological composition.

13. Emulsion according to any one of Claims 1 to 12, characterized in that it contains at least one additive chosen from hydrophilic active substances, lipophilic active substances, stabilizers, antioxidants, perfumes, fillers, sunscreens, colorants and lipid vesicles.

14. Use of the emulsion according to any of Claims 1 to 13 for the cosmetic treatment of the skin and/or hair.

15. Use of the emulsion according to any one of Claims 1 to 13 for obtaining a cream intended for the dermatological treatment of the skin.

16. Process for cosmetic treatment, characterized in that it consists in applying to the skin and/or hair the emulsion according to any one Claims 1 to 13.

17. Use of expanded hollow thermoplastic particles of acrylonitrile polymer or copolymer for dispersing, without surfactant, an oily phase in a gelled aqueous phase and for stabilizing the emulsion thus obtained.

18. Use according to Claim 17, characterized in that the particles have a particle size of 1 to 80 µm.

19. Use according to Claim 17 or 18, characterized in that the particles have a particle size of 10 to 50 µm.

20. Use according to any one of Claims 17 to 19, characterized in that the particles have a density of 15 to 200 kg/m³.

21. Use according to any one of Claims 17 to 20, characterized in that the particles have a density of 40 to 120 kg/m³.

22. Use according to any one of Claims 17 to 21, characterized in that the particles are expanded hollow particles of copolymers of acrylonitrile and of vinylidene chloride and/or of an acrylic or styrene-based monomer.

## Patentansprüche

1. Emulsion einer Ölphase in einer als Gel vorliegenden wäßrigen Phase, dadurch gekennzeichnet, daß sie expandierte hohle thermoplastische Partikel aus einem Acrylnitril-Polymer oder -Copolymer enthält, die die Dispersion der Ölphase in der wäßrigen Phase gewährleisten, und daß sie keinen grenzflächenaktiven Stoff enthält.

2. Emulsion nach Anspruch 1, dadurch gekennzeichnet, daß sie keinen einwertigen Alkohol enthält.

3. Emulsion nach Anspruch 1 oder 2, dadurch gekennzeichnet, daß die als Gel vorliegende wäßrige Phase mindestens einen Gelbildner enthält, der unter Cärboxyvinylpolymeren, Polyacrylamiden, Polyacrylaten, Polymethacrylaten, Polysacchariden, natürlichen Gummen und Tonen ausgewählt ist.

4. Emulsion nach einem der Ansprüche 1 bis 3, dadurch gekennzeichnet, daß die Partikel eine Korngröße von 1 bis 80 µm aufweisen.

5. Emulsion nach einem der Ansprüche 1 bis 4, dadurch gekennzeichnet, daß die Partikel eine Korngröße von 10 bis 50 µm aufweisen.

6. Emulsion nach einem der Ansprüche 1 bis 5, dadurch gekennzeichnet, daß die Partikel eine Dichte von 15 bis 200 kg/m³ haben.

7. Emulsion nach einem der Ansprüche 1 bis 6, dadurch gekennzeichnet, daß die Partikel eine Dichte von 40 bis 120 kg/m³ haben.

8. Emulsion nach einem der Ansprüche 1 bis 7, dadurch gekennzeichnet, daß die Partikel expandierte hohle Partikel aus Copolymeren von Acrynitril und Vinylidenchlorid und/oder einem Acrylmonomer oder Styrolmonomer sind.

9. Emulsion nach einem der Ansprüche 1 bis 8, dadurch gekennzeichnet, daß der Anteil der Partikel 0,1 bis 10 Gew.-%, bezogen auf das Gesamtgewicht der Emulsion, beträgt.

10. Emulsion nach einem der Ansprüche 1 bis 9, dadurch gekennzeichnet, daß der Anteil des Gelbildners 0,1 bis 10 Gew.-%, bezogen auf das Gesamtgewicht der Emulsion, beträgt.

11. Emulsion nach einem der Ansprüche 1 bis 10, dadurch gekennzeichnet, daß der Anteil der Ölphase 0,1 bis 30 Gew.-%, bezogen auf das Gesamtgewicht der Emulsion, beträgt.

12. Emulsion nach einem der Ansprüche 1 bis 11, dadurch gekennzeichnet, daß sie aus einer kosmetischen und/oder dermatologischen Zusammensetzung besteht.

13. Emulsion nach einem der Ansprüche 1 bis 12, dadurch gekennzeichnet, daß sie mindestens einen Zusatz enthält, der unter hydrophilen Wirkstoffen, lipophilen Wirkstoffen, Konservierungsmitteln, Antioxidantien, Parfüms, Füllstoffen, Sonnenschutzfiltern, Farbmitteln und Lipidvesikeln ausgewählt wird.

14. Verwendung der Emulsion nach einem der Ansprüche 1 bis 13 zur kosmetischen Behandlung der Haut und/oder des Haars.

15. Verwendung der Emulsion nach einem der Ansprüche 1 bis 13 zur Herstellung einer Creme, die für die dermatologische Behandlung der Haut bestimmt ist.

16. Verfahren zur kosmetischen Behandlung, dadurch gekennzeichnet, daß es darin besteht, die Emulsion nach einem der Ansprüche 1 bis 13 auf die Haut und/oder das Haar aufzutragen.

17. Verwendung der expandierten hohlen thermoplastischen Partikel aus einem Acrylnitril-Polymer oder -Copolymer zum Dispergieren einer Ölphase, ohne daß hierfür ein grenzflächenaktiver Stoff verwendet wird, in einer als Gel vorliegenden wäßrigen Phase und zum Stabilisieren der so erhaltenen Emulsion.

18. Verwendung nach Anspruch 17, dadurch gekennzeichnet, daß die Partikel eine Korngröße von 1 bis 80µm haben.

19. Verwendung nach Anspruch 17 oder 18, dadurch gekennzeichnet, daß die Partikel eine Korngröße von 10 bis 50 µm haben.

20. Verwendung nach einem der Ansprüche 17 bis 19, dadurch gekennzeichnet, daß die Partikel eine Dichte von 15 bis 200 kg/m³ haben.

21. Verwendung nach einem der Ansprüche 17 bis 20, dadurch gekennzeichnet, daß die Partikel eine Dichte von 40 bis 120 kg/m³ haben.

22. Verwendung nach einem der Ansprüche 17 bis 21, dadurch gekennzeichnet, daß die Partikel expandierte hohle Partikel aus Copolymeren von Acrylnitril und Vinylidenchlorid und/oder einem Acrylmonomer oder Styrolmonomer sind.
